(19) Europäisches Patentamt
European Patent Office
Office européen des brevets

(11) Numéro de publication: **0 161 172**
**A1**

(12) # DEMANDE DE BREVET EUROPEEN

(21) Numéro de dépôt: **85400723.4**

(22) Date de dépôt: **11.04.85**

(51) Int. Cl.[4]: **C 07 C 149/273**
C 07 D 209/20, C 07 C 149/247
A 61 K 31/195

(30) Priorité: **19.04.84 FR 8406200**
**19.04.84 FR 8406201**

(43) Date de publication de la demande:
**13.11.85 Bulletin 85/46**

(84) Etats contractants désignés:
**AT BE CH DE FR GB IT LI LU NL SE**

(71) Demandeur: **SYNTHELABO**
**58, rue de la Glacière**
**F-75621 Paris Cedex 13(FR)**

(72) Inventeur: **Comarmond, Jacques**
**10, Villa d'Este**
**F-75013 Paris(FR)**

(72) Inventeur: **Rivron, Luc**
**5, rue du Château**
**F-92600 Asnieres(FR)**

(72) Inventeur: **Purcell, Thomas**
**47Bis, rue de la Millière Les Mesnuls**
**F-78490 Montfort l'Amaury(FR)**

(74) Mandataire: **Ludwig, Jacques et al,**
**SYNTHELABO Service Brevets 58, rue de la Glacière**
**F-75621 Paris Cedex 13(FR)**

(54) **Dérivés d'acide n-(phenylmethylthio-3 oxo-1 propyl)-aminoacétique, leur préparation et leur application en thérapeutique.**

(57) Composés répondant à la formule générale I

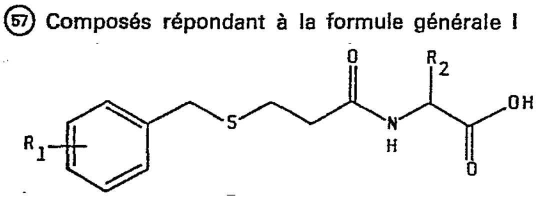

(I)

dans laquelle $R_1$ représente un ou deux atomes d'halogène, un ou deux groupes $CH_3$, un groupe $CF_3$, $OCH_3$, CN, $NO_2$, $CONH_2$, $COC_6H_5$, un noyau benzo condensé ou lorsque $R_2$ est un groupe (indolyl-3)méthyle ou $CH_2CH_2SCH_3$, un atome d'hydrogène, et $R_2$ représente un atome d'hydrogène ou un $(C_{1-4})$alkyle, un groupe $CH_2CONH_2$, $CH_2CH_2CONH_2$, $CH_2CH_2SCH_3$, $CH_2C_6H_5$, $CH_2C_6H_4OH$, ou un groupe (indolyl-3)méthyle.

EP 0 161 172 A1

La présente invention a pour objet des dérivés d'acide N-(phénylméthylthio-3 oxo-1 propyl)-aminoacétique, leur préparation et leur application en thérapeutique.

Les composés de l'invention répondent à la formule I

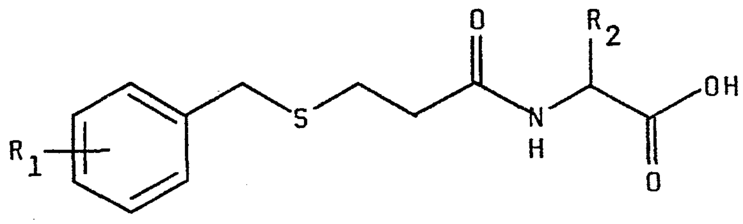

(I)

dans laquelle

$R_1$ représente un ou deux atomes d'halogène, un ou deux groupes méthyles, un groupe trifluorométhyle, méthoxy, cyano, nitro, carbamoyle, benzoyle, un noyau benzo condensé ou encore, lorsque $R_2$ est un groupe (indolyl-3)méthyle ou méthylthio-2 éthyle, $R_1$ peut représenter aussi un atome d'hydrogène, et

$R_2$ représente un atome d'hydrogène ou un groupe $(C_{1-4})$ alkyle linéaire ou ramifié, un groupe carbamoylméthyle ou carbamoyl-2 éthyle, un groupe méthylthio-2 éthyle, un groupe benzyle éventuellement hydroxylé, ou un groupe (indolyl-3)méthyle.

Les composés de l'invention peuvent exister à l'état d'acides libres ou de sels, qu'ils peuvent former avec des bases acceptables en pharmacologie.

Lorsque $R_2$ n'est pas un atome d'hydrogène, la molécule de formule I comporte un atome de carbone asymétrique. Les composés peuvent donc se présenter sous forme de deux énantiomères. Ces derniers, ainsi que leurs mélanges, font également partie de l'invention.

La préparation des composés de l'invention peut être illustrée par le schéma suivant :

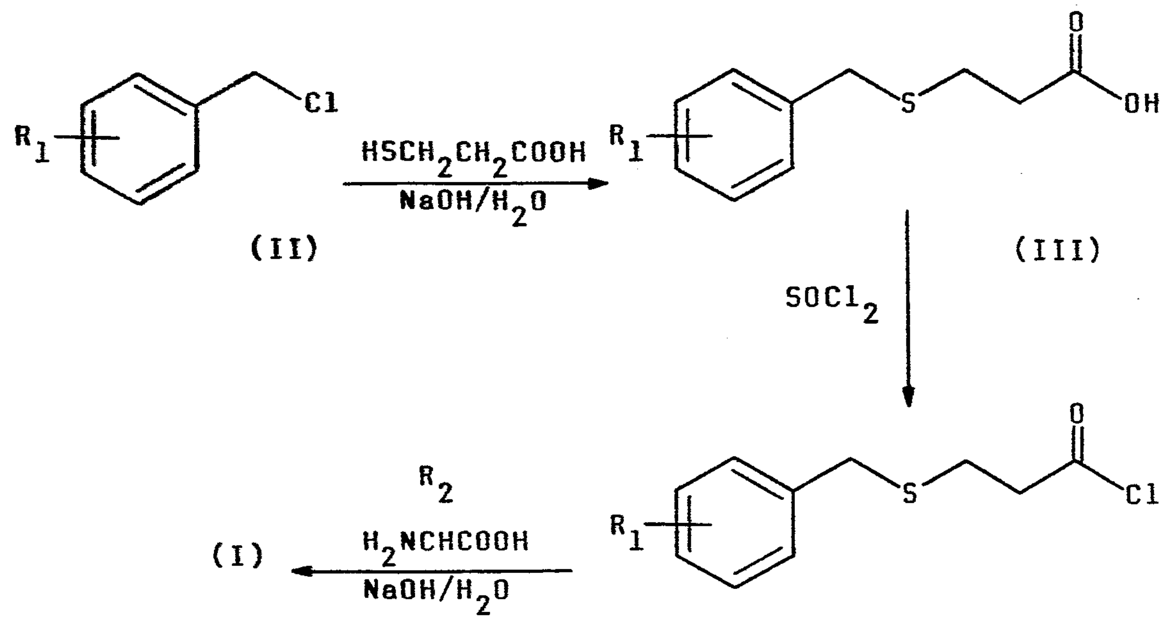


(II) (III) (IV) (I)

Le procédé consiste en trois étapes de réactions de type connu : un dérivé halogéné (II) portant le substituant $R_1$ choisi, est d'abord soumis à l'action de l'acide mercaptopropionique en milieu basique aqueux. L'acide (III) ainsi obtenu est ensuite transformé en chlorure d'acide (IV), ce dernier étant finalement soumis à l'action d'un aminoacide de formule $H_2N\underset{R_2}{C}HCOOH$ dans des conditions analogues à celles de la première étape.

Le composé (I) obtenu peut ensuite être éventuellement transformé en un autre composé (I) par modification du substituant $R_1$. Ainsi par exemple un groupe cyano peut être hydrolysé en un groupe carbamoyle.

Les exemples suivants illustrent la préparation des composés de l'invention, dont les structures sont confirmées par les analyses et les spectres IR et RMN.

Exemple 1. Acide N-[(chloro-2 phényl)méthylthio-3 oxo-1 propyl]-aminoacétique.

Dans un ballon on agite vigoureusement à 70°C un mélange de 21,2 g (0,2 mole) d'acide mercapto-3 propionique, 160 ml

d'eau, 16 g (0,4 mole) de soude et 32,2 g (0,2 mole) de chloro-1 chlorométhyl-2 benzène.
Après 15 minutes on ajoute environ 150 g de glace pilée, puis 20 ml d'acide chlorhydrique 12 N. On essore le précipité, le lave à l'eau et le sèche.
On le reprend avec un mélange de 100 ml de benzène et 21,9 ml (0,3 mole) de chlorure de thionyle, et on chauffe au reflux pendant 2 heures.
On évapore le solvant sous pression réduite et, tout en agitant vigoureusement, on verse le résidu à 0°C sur une solution préparée à partir de 100 ml d'eau, 30 g (0,4 mole) de glycine et 16 g (0,4 mole) de soude. Après 15 minutes d'agitation à une température d'environ 10°C, on dilue le mélange avec 1 volume d'eau et on ajoute 40 ml d'acide chlorhydrique 12 N. On filtre le précipité obtenu, on le lave, on le recristallise dans un mélange éthanol/eau et on le sèche.
Point de fusion : 101-102°C.

Exemple 2. Acide N-[(carbamoyl-4 phényl)-méthylthio-3 oxo-1 propyl]-aminoacétique.

a) Dans des conditions analogues à celles de l'exemple 1, on prépare d'abord l'acide N-[(cyano-4 phényl)méthylthio-3 oxo-1 propyl]-aminoacétique. Point de fusion 116-118°C.
b) Puis on dissout 12 g (0,043 mole) dans 100 ml d'acide formique et, entre 0 et 10°C, on y fait barboter du gaz chlorhydrique durant 30 minutes. On laisse ensuite reposer un jour à température ambiante à l'abri de l'humidité.
On évapore le mélange sous pression réduite, on reprend le résidu avec de l'eau, on l'essore, on le lave, on le recristallise deux fois dans le méthanol et une fois dans un mélange de diméthylformamide et d'eau, et on le sèche.
Point de fusion : 206-208°C.

Exemple 3. Acide α-[(chloro-2 phénylméthylthio)-3 oxo-1 propylamino] α-(méthyl-1 propyl) acétique.

Dans un ballon on agite vigoureusement à 70°C un mélange de 21,2 g (0,2 mole) d'acide mercapto-3 propionique, 160 ml

d'eau, 16 g (0,4 mole) de soude et 32,2 g (0,2 mole) de chloro-1 chlorométhyl-2 benzène.
Après 15 minutes on ajoute environ 150 g de glace pilée, puis 20 ml d'acide chlorhydrique 12N. On essore le précipité, on le lave à l'eau et on le sèche.
On le reprend avec un mélange de 100 ml de benzène et 21,9 ml (0,3 mole) de chlorure de thionyle, et on chauffe au reflux pendant 2 heures.
On évapore le solvant sous pression réduite et, tout en agitant vigoureusement, on verse 14,9 g du résidu, à une vitesse telle que la température ne dépasse pas 10°C, sur une solution refroidie au bain de glace et préparée à partir de 50 ml d'eau, 26,2 g (0,2 mole) de L-isoleucine et 8 g (0,2 mole) de soude. On agite le mélange pendant 10 minutes sans dépasser 10°C, puis on le dilue avec 500 ml d'eau et on ajoute 20 ml d'acide chlorhydrique concentré. On laisse reposer les cristaux formés, on les essore, on les lave à l'eau et on les essore de nouveau. Après recristallisation dans un mélange éthanol/eau, puis séchage, ils fondent à 108-109°C.

Le tableau ci-après illustre les structures et les propriétés physiques d'autres composés selon l'invention.

TABLEAU

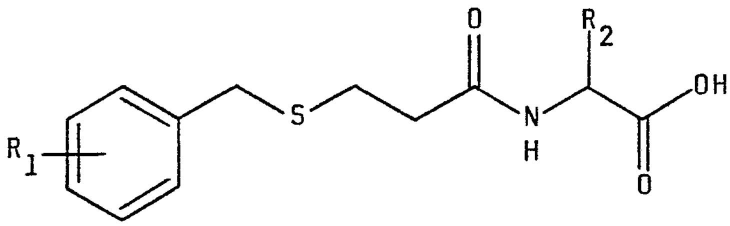

(I)

| Composé | $R_1$ | $R_2$ | F(°C) |
|---|---|---|---|
| 1(Ex. 1) | 2-Cl | H | 101-102 |
| 2 | 3-Cl | H | 105-106 |
| 3 | 4-Cl | H | 111-112 |
| 4 | 2-F | H | 77-78 |
| 5 | 3-F | H | 89-91 |
| 6 | 4-F | H | 119-120 |
| 7 | 2-Br | H | 99-100 |
| 8 | 3-Br | H | 111-113 |
| 9 | 4-Br | H | 126-127 |
| 10 | 2-I | H | 122-124 |
| 11 | 2,4-$Cl_2$ | H | 101-102 |
| 12 | 2,6-$Cl_2$ | H | 145-147 |
| 13 | 3,4-$Cl_2$ | H | 129-131 |
| 14 | 2-Cl,6-F | H | 123-124 |

| Composé | $R_1$ | $R_2$ | F(°C) |
|---|---|---|---|
| 15 | $2-CH_3$ | H | 107-108 |
| 16 | $3-CH_3$ | H | 95-96 |
| 17 | $4-CH_3$ | H | 126-127 |
| 18 | $2,5-(CH_3)_2$ | H | 105-106 |
| 19 | $3-CF_3$ | H | 100-101 |
| 20 | 2-CN | H | 127-129 |
| 21 | 3-CN | H | 109-111 |
| 22 (Ex.2a) | 4-CN | H | 116-118 |
| 23 | $2-NO_2$ | H | 115-117 |
| 24 (Ex.2b) | $4-CONH_2$ | H | 206-208 |
| 25 | $3-COC_6H_5$ | H | 91-93 |
| 26 | benzo[b] | H | 123-125 |
| 27 | $4-NO_2$ | $-CH_3$ | 88-90 |
| 28 | 2-Cl | $-CH(CH_3)_2$ | 121-122 |
| 29 | 4-F | $-CH(CH_3)_2$ | 143-145 |
| 30 | 4-F | $-CH_2CH(CH_3)_2$ | 98-100 |
| 31 | $4-NO_2$ | $-CH_2CH(CH_3)_2$ | 87-89 |
| 32 (Ex. 3) | 2-Cl | $-CH(CH_3)CH_2CH_3$ | 108-109 |

| Composé | $R_1$ | $R_2$ | F(°C) |
|---|---|---|---|
| 33 | 3-Cl | $-CH(CH_3)CH_2CH_3$ | 95-96 |
| 34 | $2-CH_3$ | $-CH(CH_3)CH_2CH_3$ | 122-123 |
| 35 | 2-Cl | $-CH_2CONH_2$ | 135-137 |
| 36 | 3-Cl | $-CH_2CONH_2$ | 162-163 |
| 37 | $4-CH_3$ | $-CH_2CONH_2$ | 156-157 |
| 38 | 2-Cl | $-CH_2CH_2CONH_2$ | 157-159 |
| 39 | H | $-CH_2CH_2SCH_3$ | 122-123 |
| 40 | 2-Cl | $-CH_2$ 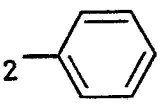 | 122-123 |
| 41 | 4-F | $-CH_2$ 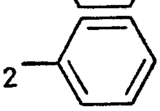 | 123-124 |
| 42 | $2-OCH_3$ | $-CH_2$ 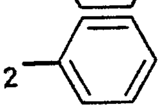 | 109-111 |
| 43 | 2-Cl | $-CH_2$ 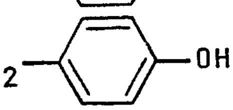 | 107-109 |
| 44 | H | $-CH_2$ 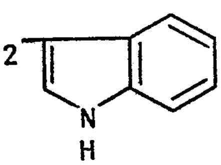 | 138-139 |
| 45 | 2-Cl | $-CH_2$ 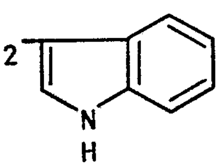 | 139-140 |
| 46 | 3-Cl | $-CH_2$ 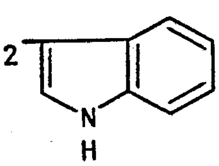 | 106-108 |

Les composés de l'invention ont fait l'objet d'essais pharmacologiques qui ont mis en évidence leur intérêt comme agents mucolytiques, anti-inflammatoires, antiulcéreux, antisécrétoires et analgésiques.

Pour l'étude de l'activité mucolytique, des essais ont été effectués sur le modèle expérimental de bronchite à l'anhydride sulfureux chez le rat, selon la méthode instaurée par L. Reid ("An experimental study of hypersecretion of mucus in the bronchial tree", Brit. J. Exp. Pathol., 1963, 44, 437) et développée par A. Quevauviller et coll. ("Méthodes d'étude expérimentale des modificateurs des sécrétions bronchiques", Le Poumon et le Coeur, 1970, 26, n° 1, 71-80).
Les animaux utilisés sont des rats mâles Sprague Dawley SPF de Charles River (France) d'un poids moyen de 300 g répartis au hasard en lots à l'aide d'une table de répartition. Ils sont soumis pendant 3 jours, à raison de 5 ou 6 heures par jour, à une inhalation d'anhydride sulfureux à une concentration de 500 ou 600 ppm. A partir du 4e jour et jusqu'au 13e jour, les animaux sont traités avec des composés de l'invention, à raison de 100 mg par kg et par jour, par voie orale. Les animaux témoins ne reçoivent que le solvant, une solution de tween 80 à 1 %.
Au cours des 8e, 9e et 11e jour, tous les animaux sont encore soumis à une inhalation de 300 ppm d'anhydride sulfureux pendant 1 ou 2 heures, tout en recevant les composés de l'invention ou le solvant.
Les animaux sont sacrifiés 24 heures après le dernier jour de traitement, par section de l'aorte abdominale après anesthésie au pentobarbital.
Après prélèvement, l'arbre trachéobronchique est fixé au formol 10 % tamponné à pH 7.

En vue de l'évaluation de la rétention trachéobronchique, le mucus est coloré par une solution de Bleu Alcian à 7°/₀₀, après coupe des bronches principales et de la trachée selon leur axe longitudinal, et durcissement préalable des tissus à l'alcool.
L'évaluation de la rétention endobronchique est effectuée au

niveau de la bronche principale du poumon gauche et du lobe basal droit, ainsi qu'au niveau de la trachée. La bronche principale et la trachée étant arbitrairement divisées en trois parties (tiers hilaire, tiers moyen et tiers distal pour la bronche ; tiers supérieur, tiers moyen et tiers inférieur pour la trachée), l'évaluation de la rétention est effectuée au niveau de chaque tiers à l'aide d'une loupe binoculaire.

La rétention de mucus dans les voies respiratoires pouvant se présenter macroscopiquement sous des aspects très différents, quatre formes principales d'obstruction sont retenues : dépôt, amas nodulaire, coulée et bouchon compact. Dans chacune de ces formes, excepté le bouchon compact, on distingue plusieurs types selon l'importance volumétrique de l'obstruction considérée.

Pour chaque animal, on estime le volume de la rétention en attribuant à chaque forme observée une valeur chiffrée correspondant au volume occupé par cette obstruction dans la lumière bronchique ou trachéale du tiers examiné. Ainsi la valeur 15 correspond à l'obstruction complète d'un bouchon compact occupant 100 % de la lumière bronchique ou trachéale et la valeur 1 correspond à l'obstruction minimum d'un petit dépôt occupant environ 1/15 de la lumière bronchique ou trachéale.

L'activité mucolytique d'un produit sur la rétention trachéobronchique de mucus pathologique est définie en fonction de la diminution globale de la rétention, c'est-à-dire du volume de mucus secrété (volume moyen par animal et volume moyen par animal et par lot).

Au niveau bronchique, le volume global de la rétention est apprécié au niveau de <u>2</u> bronches principales, soit par rapport à une cotation maximum de 90 (6 tiers bronchiques obstrués par 6 bouchons compacts : 6 x 15 = 90).

Au niveau trachéal, le volume global de la rétention n'étant apprécié que sur 3 tiers, soit par rapport à une cotation maximum de 45 (3 tiers trachéaux obstrués par 3 bouchons compacts : 3 x 15 = 45), son expression chiffrée est multipliée par 2, afin que la rétention endotrachéale et la rétention endobrochique, pour chaque animal, soient exprimées de manière comparable.

Ainsi, la valeur de la rétention trachéobronchique globale, obtenue en additionnant la valeur de la rétention endotrachéale et la valeur de la rétention endobronchique, tient compte de façon égale des deux niveaux de rétention de mucus pathologique.

Une analyse statistique des résultats est effectuée par le test non paramétrique de Mann et Whitney.

Résultats

Par rapport aux animaux témoins, les animaux traités par les composés de l'invention présentent une réduction allant jusqu'à 50 % du volume global de la rétention trachéobronchique.

Pour l'étude de l'activité anti-inflammatoire, des essais ont été effectués sur le test de l'oedème à la carragénine chez le rat selon la méthode de Winter et al. ("Carrageenin induced edema in hind paw of the rat as an assay for anti-inflammatory drugs". Proc. Soc. Exp. Biol. Med. 1962, 111, 544-547). Les animaux utilisés sont des rats mâles Sprague Dawley SPF de Charles River (France) d'un poids moyen de 150 g, répartis en lots au hasard à l'aide d'une table de répartition.

Les composés sont administrés par voie orale à des doses comprises entre 20 et 200 mg/kg, 1 heure avant l'injection sous l'aponévrose plantaire à l'une des pattes postérieures, de 0,1 ml de carragénine, en suspension à 1% dans du sérum physiologique stérile. Les animaux témoins ne reçoivent que le placebo, une solution de tween 80 à 1%. L'augmentation du volume de la patte est mesurée 3 heures après l'injection de carragénine au moyen d'un pléthysmomètre Ugo Basile.

Résultats

Par rapport aux animaux témoins, les animaux traités par les composés de l'invention présentent une diminution de 40 % du volume de l'oedème à des doses de 40 à 200 mg/kg.

Pour l'étude de l'activité anti-ulcéreuse, des essais ont été effectués vis à vis des ulcères induits, chez le rat,

par la phénylbutazone ou par l'éthanol. Les animaux utilisés sont, pour ces deux modèles, des rats femelles Wistar (Iffa Credo), à jeûn depuis 18 heures, répartis en lots au hasard à l'aide d'une table de répartition.

Les ulcères sont provoqués par l'administration orale de phénylbutazone, en solution mole à mole avec de la soude, à la dose de 200 mg/kg, ou d'éthanol à 50 %, à la dose de 5 ml/kg.

Les composés à étudier sont administrés par la voie orale 30 minutes avant l'ingestion de l'agent ulcérigène. Les animaux témoins ne reçoivent que le placebo, une solution de tween 80 à 1 %.

Les animaux sont sacrifiés, par inhalation de chloroforme, 2 heures après l'ingestion de phénylbutazone ou 1 heure après l'ingestion d'éthanol. Les estomacs sont prélevés, le degré d'ulcération est noté en aveugle de 0 à 3 et l'index d'ulcération (degré d'ulcération x pourcentage d'animaux ayant des ulcères) est calculé.

Résultats

Par rapport aux animaux témoins, les animaux traités par les composés de l'invention présentent une diminution de 50 % de l'index d'ulcération à partir de 4 mg/kg vis à vis de la phénylbutazone, et de 15 mg/kg vis à vis de l'éthanol.

Pour l'étude de l'activité anti-sécrétoire gastrique, des essais ont été effectués chez le rat à pylore ligaturé selon la technique de Shay et al. ("A simple method for the uniform production of gastric ulceration in the rat". Gastroenterology, 1945, 5, 43-61) décrite par Pascaud et Laubie ("Etude pharmacologique de thiocarboxamides anti-sécrétoires gastriques et ulcéro-protectrices". Arzneim. Forsch. 1971, 10, 1547-1553).

Les animaux utilisés sont des rats femelles Wistar (Iffa Credo) d'un poids compris entre 200 et 250 g, à jeûn depuis 48 heures et répartis en lots au hasard à l'aide d'une table de répartition. La ligature du pylore est effectuée sous légère anesthésie à l'éther après lavage de l'estomac au moyen de 4 ml de sérum physiologique tiède.

Les composés à étudier sont administrés par voie intra-péritonéale immédiatement après la ligature du pylore. Les animaux témoins ne reçoivent que le placebo, une solution de tween 80 à 1 %. 4 heures plus tard, les animaux sont sacrifiés, par inhalation de chloroforme. Le contenu gastrique est prélevé et centrifugé. Le volume de la sécrétion gastrique est mesuré, l'acidité libre et l'acidité totale sont dosées par titrimétrie.

Résultats

Par rapport aux animaux témoins, les animaux traités par les composés de l'invention présentent une diminution de 50 à 80 % de la sécrétion d'acide gastrique dès la dose de 10 mg/kg.

Pour l'étude de l'activité analgésique, des essais ont été effectués sur le "Writhing-test" à l'acide acétique chez la souris d'après Koster et al. ("Acetic acid for analgesic screening". Fed. Proc. 1959, 18, 412). Les animaux utilisés sont des souris mâles CD1 de Charles River (France), d'un poids moyen de 20 g, à jeûn depuis 18 heures. L'injection intrapéritonéale de 10 ml/kg d'une solution d'acide acétique à 0,6 % dans une solution de tween 80 à 0,2 % et de carboxyméthylcellulose à 0,25 %, déclenche en quelques minutes chez les souris un syndrôme d'étirements et de torsions (writhing) pouvant être considéré comme l'expression d'une douleur abdominale diffuse. Les étirements sont comptabilisés pendant les 15 premières minutes qui suivent cette injection.

Les composés à étudier sont administrés par voie orale, 30 minutes avant l'injection d'acide acétique. Les animaux témoins ne reçoivent que le placebo, une solution de tween 80 à 1 %.

Résultats

Par rapport aux animaux témoins, les animaux traités par les composés de l'invention présentent une diminution de 50 % du nombre des étirements provoqués par l'acide acétique à des doses de 20 à 200 mg/kg.

Les résultats ci-dessus montrent que les composés de l'invention peuvent être utilisés comme substances actives de médicaments et compositions pharmaceutiques destinés au traitement de diverses affections de l'appareil broncho-respiratoire accompagnées d'une hypersécrétion de mucus, telles que bronchites, trachéo-bronchites, bronchorrhées, asthme, etc, et, de manière générale, de toutes affections liées aux désordres de la sécrétion ou du transport du mucus, au traitement d'inflammations et de douleurs d'origines diverses, ainsi qu'au traitement des ulcères et des hypersécrétions gastriques.

A cet effet ils peuvent être présentés sous des formes appropriées à l'administration entérale ou parentérale, par exemple sous forme de comprimés, gélules, dragées, sirops, solutions ou suspensions buvables ou injectables, en association avec des excipients convenables.

La posologie quotidienne peut aller de 0,5 à 100 mg/kg de substance active, soit environ 25 à 7000 mg.

Revendications pour les états contractants :
BE, CH, DE, FR, GB, IT, LI, LU, NL, SE

1. Composés sous forme d'énantiomères ou de leurs mélanges, répondant à la formule générale I

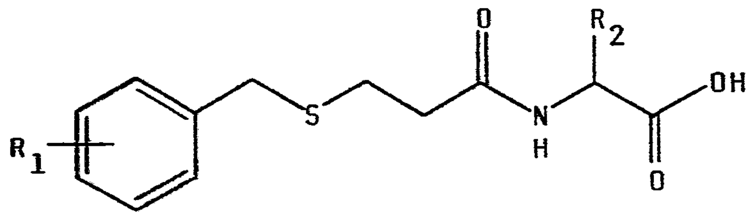

(I)

dans laquelle

$R_1$ représente un ou deux atomes d'halogène, un ou deux groupes méthyles, un groupe trifluorométhyle, méthoxy, cyano, nitro, carbamoyle, benzoyle, un noyau benzo condensé ou encore, lorsque $R_2$ est un groupe (indolyl-3)méthyle ou méthylthio-2 éthyle, $R_1$ peut représenter aussi un atome d'hydrogène, et

$R_2$ représente un atome d'hydrogène ou un groupe ($C_{1-4}$) alkyle linéaire ou ramifié, un groupe carbamoylméthyle ou carbamoyl-2 éthyle, un groupe méthylthio-2 éthyle, un groupe benzyle éventuellement hydroxylé, ou un groupe (indolyl-3)méthyle,

ainsi que leurs sels acceptables en pharmacologie.

2. Composé selon la revendication 1, caractérisé en ce que $R_1$ représente un atome de chlore en position 2.

3. Composé selon la revendication 2, caractérisé en ce que $R_2$ représente un atome d'hydrogène.

4. Procédé de préparation des composés selon la revendication 1, caractérisé en ce qu'on fait réagir un dérivé halogéné de formule II

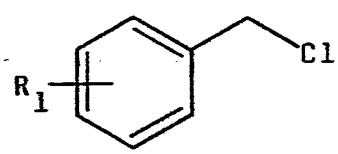

(II)

dans laquelle $R_1$ est tel que défini à la revendication 1, avec l'acide mercaptopropionique, puis on fait réagir l'acide obtenu, de formule III

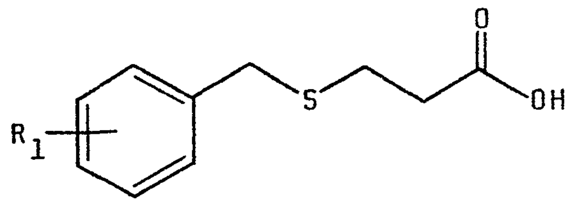

(III)

avec le chlorure de thionyle pour obtenir le chlorure d'acide de formule IV

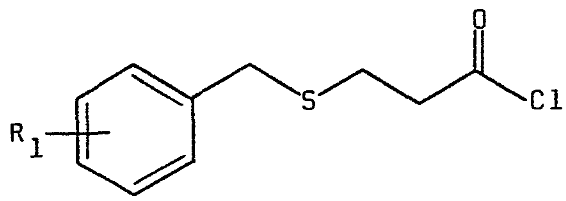

(IV)

que l'on fait réagir ensuite avec un aminoacide de formule $H_2N\underset{R_2}{\underset{|}{C}}HCOOH$, dans laquelle $R_2$ est tel que défini dans la revendication et, si nécessaire, on transforme le composé (I) ainsi obtenu en un autre composé (I) en modifiant le substituant $R_1$.

5. Médicament caractérisé en ce qu'il consiste en un composé selon l'une des revendications 1 à 3.

6. Composition pharmaceutique caractérisée en ce qu'elle contient un composé selon l'une des revendications 1 à 3, en association avec un excipient acceptable en pharmacologie.

Revendication pour l'état contractant : AT

Procédé de préparation de composés répondant à la formule générale I

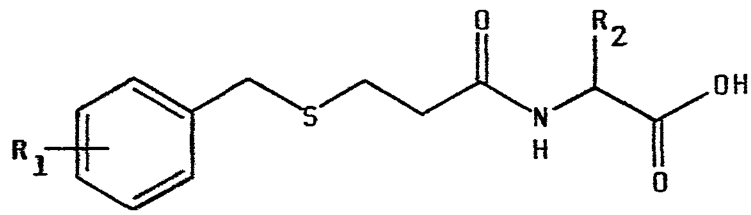

(I)

dans laquelle

$R_1$ représente un ou deux atomes d'halogène, un ou deux groupes méthyles, un groupe trifluorométhyle, méthoxy, cyano, nitro, carbamoyle, benzoyle, un noyau benzo condensé ou encore, lorsque $R_2$ est un groupe (indolyl-3)méthyle ou méthylthio-2 éthyle, $R_1$ peut représenter aussi un atome d'hydrogène, et

$R_2$ représente un atome d'hydrogène ou un groupe $(C_{1-4})$ alkyle linéaire ou ramifié, un groupe carbamoylméthyle ou carbamoyl-2 éthyle, un groupe méthylthio-2 éthyle, un groupe benzyle éventuellement hydroxylé, ou un groupe (indolyl-3)méthyle,

procédé caractérisé en ce qu'on fait réagir un dérivé halogéné de formule II

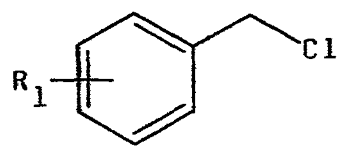

(II)

dans laquelle $R_1$ est tel que défini ci-dessus, avec l'acide mercaptopropionique, puis on fait réagir l'acide obtenu, de formule III

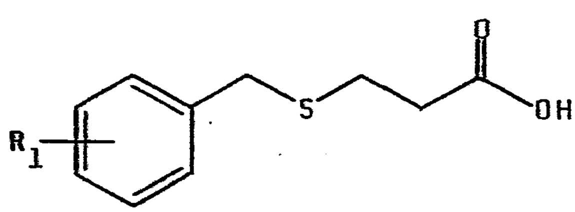

(III)

avec le chlorure de thionyle pour obtenir le chlorure

d'acide de formule IV

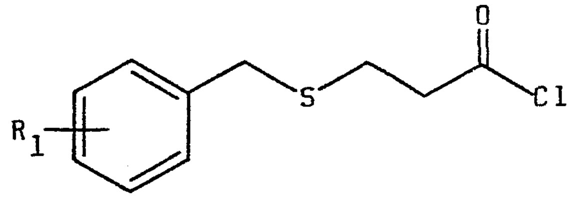

(IV)

que l'on fait réagir ensuite avec un aminoacide de formule $H_2N\underset{R_2}{\underset{|}{C}}HCOOH$, dans laquelle $R_2$ est tel que défini ci-dessus et, si nécessaire, on transforme le composé (I) ainsi obtenu en un autre composé (I) en modifiant le substituant $R_1$.

Office européen des brevets

# RAPPORT DE RECHERCHE EUROPEENNE

0161172
Numero de la demande

EP 85 40 0723

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-3 246 025 (ITARU MITAL) * Colonne 1, ligne 21 - colonne 2, ligne 21; exemples 2,5 * --- | 1,4-6 | C 07 C 149/273<br>C 07 D 209/20<br>C 07 C 149/247<br>A 61 K 31/195 |
| A | EP-A-0 013 261 (SIGMA - TAU) * Exemple * --- | 1,4 | |
| A | DE-A-2 709 820 (SANTEN PHARMACEUTICAL) * Exemples 1,2 * --- | 1-6 | |
| A | CHEMICAL ABSTRACTS, vol. 83, no. 1, 7 juillet 1975, page 916, no. 10803s, Columbus, Ohio, US; B.M. FERRIER et al.: "Synthesis and some biological properties of 1-deamino-4-glu-oxytocin (1-bêta-mercaptopropionic acid-4-glutamic acid-oxytocin) and its use in preparing a hormone-agarose complex" & CAN. J. BIOCHEM. 1975, 53(1), 21-7. Et Chemical Abstracts, 9th Coll. Index, vol. 76-85, 1972-1976, Chem. Subst. Tungstic-Z, page 39695 CS * En entier * ----- | 1-6 | DOMAINES TECHNIQUES RECHERCHES (Int. Cl.4)<br>C 07 C 149/00<br>C 07 D 209/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 24-07-1985 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503.03.82